# EUROPEAN PATENT SPECIFICATION

(11) **EP 1 021 542 B1**
(45) Date of publication and mention of the grant of the patent: **04.03.2009**
(21) Application number: 98953378.1
(22) Date of filing: 09.10.1998
(51) Int. Cl.: C12N 15/19, C07K 14/705, C12N 15/62, C07K 16/24, A61K 38/19

(54) **APO-3 LIGAND**
APO-3 LIGAND
LIGAND APO-3

(30) Priority: 10.10.1997 US 62037 P; 17.12.1997 US 69862 P
(43) Date of publication of application: 26.07.2000
(60) Divisional application: 08004651.9
(73) Proprietor: Genentech, Inc., South San Francisco CA 94080-4990 (US)
(72) Inventor: ASHKENAZI, Avi, J., San Mateo, CA 94402 (US); MARSTERS, Scot, A., San Carlos, CA 94070 (US); PITTI, Robert, El Cerrito, CA 94530 (US)
(74) Representative: Kiddle, Simon John
(86) International application number: PCT/US1998/021407
(87) International publication number: WO 1999/019490

(56) References cited:
- WO-A-97/25428
- WO-A-97/37020
- WO-A-98/05783
- WO-A-98/35061
- ASHKENAZI, A. ET AL.: "Induction of apoptosis by Apo-2 ligand, a new member of the TNF cytokine family." FASEB JOURNAL, vol. 10, no. 6, 30 April 1996, pages a1276-aBSTR.1600, XP002092398
- CHICHEPORTICHE ET AL: "TWEAK, a new secreted ligand in the tumor necrosis factor family that weakly induces apoptosis" JOURNAL OF BIOLOGICAL CHEMISTRY, vol. 272, no. 51, 19 December 1997, pages 32401-10, XP002076164
- MARSTERS, S.A. ET AL.: "Identification of a ligand for the death-domain-containing receptor Apo3." CURRENT BIOLOGY, vol. 8, no. 9, 23 April 1998, pages 525-8, XP002092399

### SEQUENCE LISTING

<110> GENENTECH INC.
<120> APO-3 LIGAND POLYPEPTIDE
<130> 11669.27WO01
<140> NEW FILING
   <141> 1998-10-09
<150> 60/062,037
   <151> 1997-10-10
<150> 60/069,862
   <151> 1997-12-17
<160> 10
<170> PatentIn Ver. 2.0
<210> 1
   <211> 249
   <212> PRT
   <213> Homo sapiens
<400> 1
   Met Ala Ala Arg Arg Ser Gln Arg Arg Arg Gly Arg Arg Gly Glu Pro
     1               5                  10                  15
   Gly Thr Ala Leu Leu Val Pro Leu Ala Leu Gly Leu Gly Leu Ala Leu
                20                  25                  30
   Ala Cys Leu Gly Leu Leu Leu Ala Val Val Ser Leu Gly Ser Arg Ala
            35                  40                  45
   Ser Leu Ser Ala Gln Glu Pro Ala Gln Glu Glu Leu Val Ala Glu Glu
        50                  55                  60
   Asp Gln Asp Pro Ser Glu Leu Asn Pro Gln Thr Glu Glu Ser Gln Asp
    65                  70                  75                  80
   Pro Ala Pro Phe Leu Asn Arg Leu Val Arg Pro Arg Arg Ser Ala Pro
                    85                  90                  95
   Lys Gly Arg Lys Thr Arg Ala Arg Arg Ala Ile Ala Ala His Tyr Glu
               100                 105                 110
   Val His Pro Arg Pro Gly Gln Asp Gly Ala Gln Ala Gly Val Asp Gly
           115                 120                 125
   Thr Val Ser Gly Trp Glu Glu Ala Arg Ile Asn Ser Ser Ser Pro Leu
       130                 135                 140
   Arg Tyr Asn Arg Gln Ile Gly Glu Phe Ile Val Thr Arg Ala Gly Leu
   145                 150                 155                 160
   Tyr Tyr Leu Tyr Cys Gln Val His Phe Asp Glu Gly Lys Ala Val Tyr
                   165                 170                 175
   Leu Lys Leu Asp Leu Leu Val Asp Gly Val Leu Ala Leu Arg Cys Leu
               180                 185                 190
   Glu Glu Phe Ser Ala Thr Ala Ala Ser Ser Leu Gly Pro Gln Leu Arg
           195                 200                 205
   Leu Cys Gln Val Ser Gly Leu Leu Ala Leu Arg Pro Gly Ser Ser Leu
       210                 215                 220
   Arg Ile Arg Thr Leu Pro Trp Ala His Leu Lys Ala Ala Pro Phe Leu
   225                 230                 235                 240
   Thr Tyr Phe Gly Leu Phe Gln Val His
                   245
<210> 2
   <211> 1421
   <212> DNA
   <213> Homo sapiens
<400> 2
   tctagagatc cctcgacctc gacccacgcg tccgcgatcc ctcgggtccc gggatggggg 60
   ggcggtgagg caggcacagc cccccgcccc catggccgcc cgtcggagcc agaggcggag 120
   ggggcgccgg ggggagccgg gcaccgccct gctggtcccg ctcgcgctgg gcctgggcct 180
   ggcgctggcc tgcctcggcc tcctgctggc cgtggtcagt ttggggagcc gggcatcgct 240
   gtccgcccag gagcctgccc aggaggagct ggtggcagag gaggaccagg acccgtcgga 300
   actgaatccc cagacagaag aaagccagga tcctgcgcct ttcctgaacc gactagttcg 360
   gcctcgcaga agtgcaccta aaggccggaa aacacgggct cgaagagcga tcgcagccca 420
   ttatgaagtt catccacgac ctggacagga cggagcgcag gcaggtgtgg acgggacagt 480
   gagtggctgg gaggaagcca gaatcaacag ctccagccct ctgcgctaca accgccagat 540
   cggggagttt atagtcaccc gggctgggct ctactacctg tactgtcagg tgcactttga 600
   tgaggggaag gctgtctacc tgaagctgga cttgctggtg gatggtgtgc tggccctgcg 660
   ctgcctggag gaattctcag ccactgcggc gagttccctc gggccccagc tccgcctctg 720
   ccaggtgtct gggctgttgg ccctgcggcc agggtcctcc ctgcggatcc gcaccctccc 780
   ctgggcccat ctcaaggctg cccccttcct cacctacttc ggactcttcc aggttcactg 840
   aggggccctg gtctccccgc agtcgtccca ggctgccggc tcccctcgac agctctctgg 900
   gcacccggtc ccctctgccc caccctcagc cgctctttgc tccagacctg cccctccctc 960
   tagaggctgc ctgggcctgt tcacgtgttt tccatcccac ataaatacag tattcccact 1020
   cttatcttac aactccccca ccgcccactc tccacctcac tagctcccca atccctgacc 1080
   ctttgaggcc cccagtgatc tcgactcccc cctggccaca gacccccagg tcattgtgtt 1140
   cactgtactc tgtgggcaag gatgggtcca gaagacccca cttcaggcac taagaggggc 1200
   tggacctggc ggcaggaagc caaagagact gggcctaggc caggagttcc caaatgtgag 1260
   gggcgagaaa caagacaagc tcctcccttg agaattccct gtggattttt aaaacagata 1320
   ttatttttat tattattgtg acaaaatgtt gataaatgga tattaaatag aataagtcat 1380
   aaaaaaaaaa aaaaaaaaaa aagggcggcc gcgactctag a 1421
<210> 3
   <211> 254
   <212> DNA
   <213> Unknown
<220>
   <223> Description of Unknown Organism:Unknown
<400> 3
   gattttttaa gttacggtct ggagaggaaa tcagcatcga ggtctccaac ccctccttac 60
   tggatccgga tcaggatgca acatactttg gggcttttaa agttcgagat atagattgag 120
   ccccagtttt tggagtgtta tgtatttcct ggatgtttgg aaacattttt taaaacaagc 180
   caagaaagat gtatataggt gtgtgagact actaagaggc atggccccaa cggtacacga 240
   ctcagtatcc atgc 254
<210> 4
   <211> 50
   <212> DNA
   <213> Unknown
<220>
   <223> Description of Unknown Organism:Unknown
<400> 4
   ccagccctct gcgctacaac cgccagatcg gggagtttat agtcacccgg 50
<210> 5
   <211> 40
   <212> DNA
   <213> Homo sapiens
<400> 5
   cgacgacaag catatgcggg catcgctgtc cgcccaggag 40
<210> 6
   <211> 38
   <212> DNA
   <213> Homo sapiens
<400> 6
   cagccggatc ctcgagtcag tgaacctgga agagtccg 38
<210> 7
   <211> 24
   <212> PRT
   <213> Unknown
<220>
   <223> Description of Unknown Organism:Unknown
<400> 7
   Met Gly His His His His His His His His His His Ser Ser Gly His
    1               5                  10                  15
   Ile Asp Asp Asp Asp Lys His Met
               20
<210> 8
   <211> 29
   <212> DNA
   <213> Unknown
<220>
   <223> Description of Unknown Organism:Unknown
<400> 8
   atcagggact ttccgctggg gactttccg 29
<210> 9
   <211> 24
   <212> DNA
   <213> Homo sapiens
<400> 9
   ccgcagtcgt cccaggctgc cggc 24
<210> 10
   <211> 23
   <212> DNA
   <213> Homo sapiens
<400> 10
   ggagctagtg aggtggagat ggg 23

## Description

### FIELD OF THE INVENTION

The present invention relates generally to the identification and isolation of novel DNA and to the recombinant production of novel polypeptides, designated herein as "Apo-3 Ligand".

### BACKGROUND OF THE INVENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, *e.g.*, Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)]. Decreased levels of apoptotic cell death have been associated with a variety of pathological conditions, including cancer, lupus, and herpes virus infection [Thompson, Science, 267:1456-1462 (1995)]. Increased levels of apoptotic cell death may be associated with a variety of other pathological conditions, including AIDS, Alzheimer's disease, Parkinson's disease, amyotrophic lateral sclerosis, multiple sclerosis, retinitis pigmentosa, cerebellar degeneration, aplastic anemia, myocardial infarction, stroke, reperfusion injury, and toxin-induced liver disease [see, Thompson, supra].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, supra; Sachs et al., Blood, 82:15 (1993)]. For instance, they can be triggered by hormonal stimuli, such as glucocorticoid hormones for immature thymocytes, as well as withdrawal of certain growth factors [Watanabe-Fukunaga et al., Nature, 356:314-317 (1992)]. Also, some identified oncogenes such as *myc, rel,* and *E1A*, and tumor suppressors, like *p53*, have been reported to have a role in inducing apoptosis. Certain chemotherapy drugs and some forms of radiation have likewise been observed to have apoptosis-inducing activity (Thompson, supra].

Various molecules, such as tumor necrosis factor-α ("TNF-α"), tumor necrosis factor-β ("TNF-β" or "lymphotoxin-α"), lymphotoxin-β ("LT-β"), CD30 ligand, CD27 ligand, CD40 ligand, OX-40 ligand, 4-1BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood, 85:3378-3404 (1995); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992)]. Among these molecules, TNF-α, TNF-β, CD30 ligand, 4-1BB ligand, Apo-1 ligand, and Apo-2 ligand (TRAIL) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)]. Zheng et al. have reported that TNF-α is involved in post-stimulation apoptosis of CD8-positive T cells [Zheng et al., Nature, 377:348-351 (1995)]. Other investigators have reported that.CD30 ligand may be involved in deletion of self-reactive T cells in the thymus [Amakawa et al., Cold Spring Harbor Laboratory Symposium on Programmed Cell Death, Abstr. No. 10, (1995)].

Mutations in the mouse Fas/Apo-1 receptor or ligand genes (called *lpr* and *gld*, respectively) have been associated with some autoimmune disorders, indicating that Apo-1 ligand may play a role in regulating the clonal deletion of self-reactive lymphocytes in the periphery [Krammer et al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified [Hohman et al., J. Biol. Chem., 264:14927-14934 (1989); Brockhaus et al., Proc. Natl. Acad. Sci., 87:3127-3131.(1990); EP 417,563, published March 20, 1991] and human and mouse cDNAs corresponding to both receptor types have been isolated and characterized [Loetscher et al., Cell, 61: (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al.; Proc. Natl. Acad. Sci. U.S.A., 87:8331 (1990)]. More recently, the cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra] . In TNFR1, the approximate boundaries of the four CRDs are as follows: CRD1-amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao, M.V., J. Biol. Chem., 266:12099-12104 (1991)] had little or no effect on NGF binding [Yan, H. and Chao, M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

Itoh et al. disclose that the Apo-1 receptor can signal an apoptotic cell death similar to that signaled by the 55-kDa TNFR1 [Itoh et al., supra]. Expression of the Apo-1 antigen has also been reported to be down-regulated along with that of TNFR1 when cells are treated with either TNF-α or anti-Apo-1 mouse monoclonal antibody [Krammer et al., supra; Nagata et al., supra]. Accordingly, some investigators have hypothesized that cell lines that co-express both Apo-1 and TNFR1 receptors may mediate cell killing through common signaling pathways [Id.].

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al., Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence.

In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1 and TRAMP (Chinnaiyan et al., Science, 274: 990 (1996); Kitson et al., Nature, 384:372 (1996); Bodmer et al., Immunity, 6:79 (1997)].

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-821 (1997) and Pan et al., Science, 277: 815-818 (1997), another molecule believed to be a receptor for the Apo-2 ligand (TRAIL) is described. That molecule is referred to as DR5 (it has also been alternatively referred to as Apo-2). Like DR4, DR5 is reported to contain a cytoplasmic death domain and be capable of signaling apoptosis.

In Sheridan et al., supra, a receptor called DcR1 (or alternatively, Apo-2DcR) is disclosed as being a potential decoy receptor for Apo-2 ligand (TRAIL). Sheridan et al. report that DcR1 can inhibit Apo-2 ligand function *in vitro*. See also, Pan et al., supra, for disclosure on the decoy receptor referred to as TRID.

For a review of the TNF family of cytokines and their receptors, see Gruss and Dower, supra.

As presently understood, the cell death program contains at least three important elements - activators, inhibitors, and effectors; in *C. elegans,* these elements are encoded respectively by three genes, *Ced-4, Ced-9* and *Ced*-3 [Steller, Science, 267:1445 (1995); Chinnaiyan et al., Science, 275:1122-1126 (1997); Wang et al., Cell, 90:1-20 (1997)]. Two of the TNFR family members, TNFR1 and Fas/Apo1 (CD95), can activate apoptotic cell death [Chinnaiyan and Dixit, Current Biology, 6:555-562 (1996); Fraser and Evan, Cell; 85:781-784 (1996)]. TNFR1 is also known to mediate activation of the transcription factor, NF-κB [Tartaglia et al., Cell, 74:845-853 (1993); Hsu et al., Cell, 84:299-308 (1996)]. In addition to some ECD homology, these two receptors share homology in their intracellular domain (ICD) in an oligomerization interface known as the death domain [Tartaglia et al., supra; Nagata, Cell, 88:355 (1997)]. Death domains are also found in several metazoan proteins that regulate apoptosis, namely, the Drosophila protein, Reaper, and the mammalian proteins referred to as FADD/MORT1, TRADD, and RIP [Cleaveland and Ihle, Cell, 81:479-482 (1995)]. Upon ligand binding and receptor clustering, TNFR1 and CD95 are believed to recruit FADD into a death-inducing signalling complex. CD95 purportedly binds FADD directly, while TNFR1 binds FADD indirectly via TRADD [Chinnaiyan et al., Cell, 81:505-512 (1995); Boldin et al., J. Biol. Chem., 270:387-391 (1995); Hsu et al., supra; Chinnaiyan et al:, J. Biol. Chem., 271:4961-4965 (1996)]. It has been reported that FADD serves as an adaptor protein which recruits the *Ced*-*3-*related protease, MACHα/FLICE (caspase 8), into the death signalling complex [Boldin et al., Cell, 85:803-815 (1996); Muzio et al., Cell, 85:817-827 (1996)]. MACHα/FLICE appears to be the trigger that sets off a cascade of apoptotic proteases, including the interleukin-1β converting enzyme (ICE) and CPP32/Yama, which may execute some critical aspects of the cell death programme [Fraser and Evan, supra].

It was recently disclosed that programmed cell death involves the activity of members of a family of cysteine proteases related to the *C. elegans* cell death gene, *ced-3*, and to the mammalian IL-1-converting enzyme, ICE. The activity of the ICE and CPP32/Yama proteases can be inhibited by the product of the cowpox virus gene, *crmA* [Ray et al., Cell, 69:597-604 (1992); Tewari et al., Cell, 81:801-809 (1995)]. Recent studies show that CrmA can inhibit TNFR1- and CD95-induced cell death [Enari et al., Nature, 375:78-81 (1995); Tewari et al., J. Biol. Chem., 270:3255-3260 (1995)].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-κB [Tewari et al., Curr. Op. Genet. Develop., 6:39-44 (1996)]. NF-κB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-κB is complexed with members of the IκB inhibitor family; upon inactivation of the IκB in response to certain stimuli, released NF-κB translocates to the nucleus where it binds to specific DNA sequences and activates gene transcription.

### SUMMARY OF THE INVENTION

Applicants have identified a cDNA clone that encodes a novel polypeptide designated in this specification as "Apo-3 Ligand." Apo-3 ligand is the same molecule previously referred to by Applicants as "DNA30879."

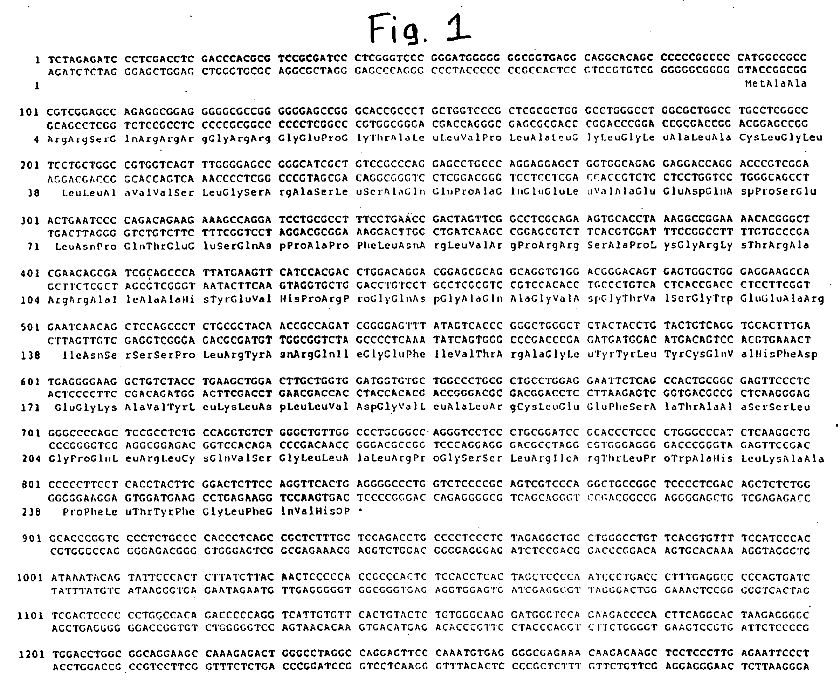

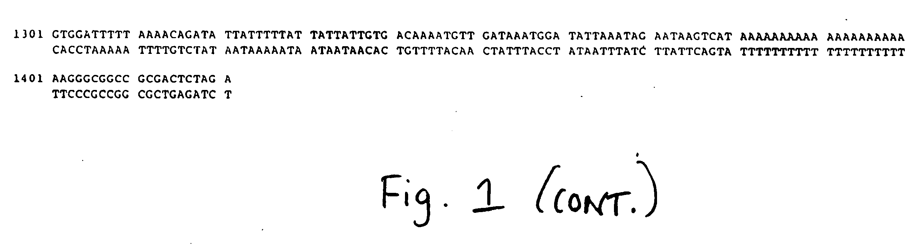

The present invention provides an isolated Apo-3 Ligand polypeptide consisting of amino acid residues 42 to 249, 47 to 249 or 52 to 249 of Fig. 1 (SEQ ID NO: 1).

The invention further provides an isolated nucleic acid molecule comprising DNA encoding said Apo-3 Ligand polypeptide.

In another embodiment, the invention provides a vector comprising said nucleic acid. A host cell comprising such a vector is also provided. By way of example, the host cells may be CHO cells, *E. coli*, or yeast. A process for producing Apo-3 Ligand polypeptides is further provided and comprises culturing host cells under conditions suitable for expression of Apo-3 Ligand and recovering Apo-3 Ligand from the cell culture.

In another embodiment, the invention provides chimeric molecules comprising said Apo-3 Ligand polypeptide fused to a heterologous polypeptide or amino acid sequence. An example of such a chimeric molecule comprises the Apo-3 Ligand fused to an epitope tag sequence or a Fc region of an immunoglobulin.

In another embodiment, the invention provides methods of using the Apo-3 Ligand polypeptide. Included in such methods are methods of inducing apoptosis in mammalian cells using the Apo-3 Ligand polypeptide.

### BRIEF DESCRIPTION OF THE DRAWINGS

Figure 1 shows the nucleotide sequence (SEQ ID NO:2) of a cDNA for human Apo-3 Ligand and its derived amino acid sequence (SEQ ID NO:1).
Figure 2 shows an EST sequence (SEQ ID NO:3) discussed in Example 1.
Figure 3A shows an alignment and comparison of the full length sequences of Apo-3 Ligand polypeptide (Apo-3L), human lymphotoxin-beta (hLTb) and human CD40 ligand (hCD40L).
Figure 3B shows an alignment and comparison of the C-terminal sequences of Apo-3 Ligand polypeptide (Apo3L), TNF-alpha (TNF), hCD40L, hLTb, Apo-2L, CD95L and LT-alpha (LTa). Residues conserved in two or more ligands are shaded. Regions that form beta-strands in the crystal structures of TNF-alpha and LT-alpha are marked by overhead lines.
Figure 4 shows expression of Apo-3 Ligand mRNA in human fetal and adult tissues and cell lines as analyzed by Northern hybridization.
Figure 5 shows NF-κB activation by Apo-3L in HeLa cells (5A) and 293 cells (5B and 5C).
Figure 6 is a bar graph showing apoptotic activity in HeLa cells transfected with Apo-3 Ligand. The increased apoptotic activity was blocked when the cells were incubated with the caspase inhibitor, z-VAD-fmk.
Figure 7 shows transfection of 293 cells with Apo-3 Ligand resulted in increased JNK/SAPK activity.
Figure 8A shows a Western blot of co-immunoprecipitated ligands/receptors. The data shows that Apo-3L specifically binds to Apo-3.
Figure 8B is a bar graph showing apoptotic activity of Apo-3L and specific complex formation between Apo-3L and Apo-3.
Figure 9A shows Apo-3L induced apoptosis in MCF-7 cells (pre-treated with CHX) as measured by phase and fluorescence microscopy.
Figure 9B shows the apoptotic effect of combined treatment of MCF-7 cells with Apo-3L and CHX.
Figure 9C shows DNA fragmentation analysis of HeLa S3 treated with Apo-3L or Apo-3L plus DEVD-fmk or zVAD-fmk.
Figure 9D shows the results of transfection of MCF-7 cells with a caspase inhibitor (p35 or CrmA) or FADD mutant followed by incubation with CHX and Apo-3L. The assay shows the transfection with a dominant-negative mutant of FADD prevented apoptosis induction by Apo-3L.

### DETAILED DESCRIPTION OF THE PREFERRED EMBODIMENTS

### I. Definitions

The terms "Apo-3 Ligand polypeptide", "Apo-3 Ligand", and "Apo-3L" when used herein encompass native sequence Apo-3 Ligand and Apo-3 Ligand variants (which are further defined herein). The Apo-3 Ligand may be isolated from a variety of sources, such as from human tissue types or from another source, or prepared by recombinant or synthetic methods.

A "native sequence Apo-3 Ligand" comprises a polypeptide having the same amino acid sequence as an Apo-3 Ligand derived from nature. Such native sequence Apo-3 Ligand can be isolated from nature or can be produced by recombinant or synthetic means. The term "native sequence Apo-3 Ligand" specifically encompassed naturally-occurring truncated or secreted forms of the Apo-3 Ligand (*e.g.*, soluble forms containing for instance, an extracellular domain sequence), naturally-occurring variant forms (*e.g.*, alternatively spliced forms) and naturally-occurring allelic variants of the Apo-3 Ligand. Mature or full-length native sequence Apo-3 Ligand polypeptide comprises amino acids 1 to 249 of Fig. 1 (SEQ ID NO:1). Apo-3 Ligand polypeptide is obtainable by expressing the polypeptide encoded by the cDNA insert of the vector deposited.as ATCC 209358.

The "Apo-3 Ligand extracellular domain" or "Apo-3 Ligand ECD" refers to a form of Apo-3 Ligand which is essentially free of the transmembrane and cytoplasmic domains of Apo-3 Ligand. Ordinarily, Apo-3 Ligand ECD will have less than 1% of such transmembrane and/or cytoplasmic domains and preferably, will have less than 0.5% of such domains. For example, Apo-3 Ligand ECD may comprise amino acid residues 47 to 249 of Fig. 1 (SEQ ID NO:1). It will be understood by the skilled artisan that the transmembrane domain identified for the Apo-3 Ligand polypeptide of Fig.1 (SEQ ID NO:1) is identified pursuant to criteria routinely employed in the art for identifying that type of hydrophobic domain. The exact boundaries of a transmembrane domain may vary but most likely by no more than about 5 amino acids at either end of the domain specifically mentioned herein. Accordingly, the Apo-3 Ligand ECD may comprise amino acids X to 249 of Figure 1 (SEQ ID NO:1) wherein X is any one of amino acid residues 42 to 52 of Figure 1 (SEQ ID NO:1).

"Percent (%) amino acid sequence identity" with respect to the Apo-3 Ligand sequences identified herein is defined as the percentage of amino acid residues in a candidate sequence that are identical with the amino acid residues in the Apo-3 Ligand sequence, after-aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared.

"Percent (%) nucleic acid sequence identity" with respect to the Apo-3 Ligand sequences identified herein is defined as the percentage of nucleotides in a candidate sequence that are identical with the nucleotides in the Apo-3 Ligand sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared.

The term "epitope tagged" when used herein refers to a chimeric polypeptide comprising Apo-3 Ligand, or a domain sequence thereof, fused to a "tag polypeptide". The tag polypeptide has enough residues to provide an epitope against which an antibody can be made, or which can be identified by some other agent, yet is short enough such that it does not interfere with activity of the Apo-3 Ligand. The tag polypeptide preferably also is fairly unique so that the antibody does not substantially cross-react with other epitopes. Suitable tag polypeptides generally have at least six amino acid residues and usually between about 8 to about 50 amino acid residues (preferably, between about 10 to about 20 residues).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in situ within recombinant cells, since at least one component of the Apo-3 Ligand natural environment will not be present. Ordinarily, however, isolated polypeptide will be prepared by at least one purification step.

An "isolated" Apo-3 Ligand nucleic acid molecule is a nucleic acid molecule that is identified and separated from at least one contaminant nucleic acid molecule with which it is ordinarily associated in the natural source of the Apo-3 Ligand nucleic acid. An isolated Apo-3 Ligand nucleic acid molecule is other than in the form or setting in which it is found in nature. Isolated Apo-3 Ligand nucleic acid molecules therefore are distinguished from the Apo-3 Ligand nucleic acid molecule as it exists in natural cells. However, an isolated Apo-3 Ligand nucleic acid molecule includes Apo-3 Ligand nucleic acid molecules contained in cells that ordinarily express Apo-3 Ligand where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers single anti-Apo-3 Ligand monoclonal antibodies (including agonist, antagonist, and neutralizing antibodies) and anti-Apo-3 Ligand antibody compositions with polyepitopic specificity. The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Biologically active" and "desired biological activity" for the purposes herein mean having the ability to modulate apoptosis (either in an agonistic or stimulating manner or in an antagonistic or blocking manner) in at least one type of mammalian cell *in vivo* or *ex vivo.*

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, all of which are known in the art.

The terms "treating," "treatment," and "therapy" as used herein refer to curative therapy, prophylactic therapy, and preventative therapy.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, blastoma, gastrointestinal cancer, renal cancer, pancreatic cancer, glioblastoma, neuroblastoma, cervical cancer, ovarian cancer, liver cancer, stomach cancer, bladder cancer, hepatoma, breast cancer, colon cancer, colorectal cancer, endometrial cancer, salivary gland cancer, kidney cancer, prostate cancer, vulval cancer, thyroid cancer, hepatic carcinoma, and various types of head and neck cancer.

The term "mammal" as used herein refers to any mammal classified as a mammal, including humans, cows, horses, dogs and cats. In a preferred embodiment of the invention, the mammal is a human.

### II. Compositions and Methods

### A. Full-length Apo-3 Ligand

Applicants have identified and isolated cDNA encoding an Apo-3 Ligand polypeptide, as disclosed in further detail in the Examples below. Using BLAST and FastA sequence alignment computer programs, Applicants found that a full-length native sequence Apo-3 Ligand (shown in Figure 1 and SEQ ID NO:1) has 23.4% amino acid sequence identity with human lymphotoxin-beta, and 19.8% amino acid sequence identity with CD40 ligand, and significant but lower identity to other members of the TNF cytokine family. As shown in the Examples below, Apo-3 Ligand polypeptide (full length and soluble forms) was found to have apoptotic activity.

### B. Modifications of Apo-3 Ligand

Covalent modifications of Apo-3 Ligand are included within the scope of this invention. One type of covalent modification includes reacting targeted amino acid residues of the Apo-3 Ligand with an organic derivatizing agent that is capable of reacting with selected side chains or the N- or C- terminal residues of the Apo-3 Ligand. Derivatization with bifunctional agents is useful, for instance, for crosslinking Apo-3 Ligand to a water-insoluble support matrix or surface for use in the method for purifying anti-Apo-3 Ligand antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis-(succinimidylpropionate); bifunctional maleimides such as bis-N-maleimido-1,8-octane and agents such as methyl-3-[(p-azidophenyl)-dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the Apo-3 Ligand polypeptide included within the scope of this invention comprises altering the native glycosylation pattern of the polypeptide. "Altering the native glycosylation pattern" is intended for purposes herein to mean deleting one or more carbohydrate moieties found in native sequence Apo-3 Ligand, and/or adding one or more glycosylation sites that are not present in the native sequence Apo-3 Ligand.

Addition of glycosylation sites to the Apo-3 Ligand polypeptide may be accomplished by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the Apo-3 Ligand polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of Apo-3 Ligand comprises linking the Apo-3 Ligand polypeptide to one of a variety of nonproteinaceous polymers, *e.g.*, polyethylene glycol, polypropylene glycol, or polyoxyalkylenes, in the manner set forth in U.S. Patent Nos. 4, 640, 835; 4, 496, 689; 4, 301, 144; 4, 670, 417; 4, 791, 192 or 9, 179, 337.

The Apo-3 Ligand of the present invention may also be modified in a way to form a chimeric molecule comprising Apo-3 Ligand fused to another, heterologous polypeptide or amino acid sequence. In one embodiment, such a chimeric molecule comprises a fusion of the Apo-3 Ligand with a tag polypeptide which provides an epitope to which an anti-tag antibody can selectively bind. The epitope tag is generally placed at the amino- or carboxyl- terminus of the Apo-3 Ligand. The presence of such epitope-tagged forms of the Apo-3 Ligand can be detected using an antibody against the tag polypeptide. Also, provision of the epitope tag enables the Apo-3 Ligand to be readily purified by affinity purification using an anti-tag antibody or another type of affinity matrix that binds to the epitope tag. In an alternative embodiment, the chimeric molecule may comprise a fusion of the Apo-3 Ligand with an immunoglobulin or a particular region of an immunoglobulin. For a bivalent form of the chimeric molecule, such a fusion could be to the Fc region of an IgG molecule. In particular, the chimeric molecule may comprise a Apo-3 Ligand which includes amino acids 47 to 249 of Fig. 1 (SEQ ID NO:1) fused to a His-tag molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1209-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

The Apo-3 Ligand of the invention may also be modified in a way to form a chimeric molecule comprising Apo-3 Ligand fused to a leucine zipper. Various leucine zipper polypeptides have been described in the art. See, e.g., Landschulz et al., Science, 240:1759 (1988); WO 94/10308; Hoppe et al., FEBS Letters, 344:1991 (1994); Maniatis et al., Nature, 341:24 (1989). It is believed that use of a leucine zipper fused to Apo-3 Ligand may be desirable to assist in dimerizing or trimerizing soluble Apo-3 Ligand in solution. Those skilled in the art will appreciate that the leucine zipper may be fused at either the 5' or 3' end of the Apo-3 Ligand molecule.

### C. Preparation of Apo-3 Ligand

The description below relates primarily to production of Apo-3 Ligand by culturing cells transformed or transfected with a vector containing Apo-3 Ligand nucleic acid. It is, of course, contemplated that alternative methods, which are well known in the art, may be employed to prepare Apo-3 Ligand. For instance, the Apo-3 Ligand sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2199-2154 (1963)]. In vitro protein synthesis may be performed using manual techniques or by automation. Automated synthesis may be accomplished, for instance, using an Applied Biosystems Peptide Synthesizer (Foster City, CA) using manufacturer's instructions. Various portions of the Apo-3 Ligand may be chemically synthesized separately and combined using chemical or enzymatic methods to produce the full-length Apo-3 Ligand.

### 1. Isolation of DNA Encoding Apo-3 Ligand

DNA encoding Apo-3 Ligand may be obtained from a cDNA library prepared from tissue believed to possess the Apo-3 Ligand mRNA and to express it at a detectable level. Accordingly, human Apo-3 Ligand DNA can be conveniently obtained from a cDNA library prepared from human tissue, such as described in the Examples. The Apo-3 Ligand-encoding gene may also be obtained from a genomic library or by oligonucleotide synthesis.

Libraries can be screened with probes (such as antibodies to the Apo-3 Ligand or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding Apo-3 Ligand is to use PCR methodology [Sambrook et al., supra; Dieffenbach et al., PCR Primer : A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined through sequence alignment using computer software programs such as ALIGN, DNAstar, and INHERIT.

Nucleic acid having protein coding sequence may be obtained by screening selected cDNA or genomic libraries using the deduced amino acid sequence disclosed herein for the first time, and, if necessary, using conventiohal primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for Apo-3 Ligand production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of transfection are known to the ordinarily skilled artisan, for example, CaPO₄ and electroporation. Depending on the host cell used, transformation is performed using standard techniques appropriate to such cells. The calcium treatment employing calcium chloride, as described in Sambrook et al., supra, or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transformations have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g.*, polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635).

In addition to prokaryotes, eukaryotic microbes such as filamentous fungi or yeast are suitable cloning or expression hosts for Apo-3 Ligand-encoding vectors. *Saccharomyces cerevisiae* is a commonly used lower eukaryotic host microorganism.

Suitable host cells for the expression of glycosylated Apo-3 Ligand are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36: 59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (*e.g.*, cDNA or genomic DNA) encoding Apo-3 Ligand may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The Apo-3 Ligand may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the Apo-3 Ligand DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, *e.g.*, the yeast invertase leader, alpha factor leader (including *Saccharomyces* and *Kluyveromyces* α-factor leaders, the latter described in U.S. Patent No. 5, 010, 182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, or (c) supply critical nutrients not available from complex media, *e.g.*, the gene encoding D-alanine racemase for *Bacilli.*

An example of suitable selectable markers for mammalian cells are those that enable the identification of cells competent to take up the Apo-3 Ligand nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain-of yeast lacking the ability to grow in tryptophan, for example, ATCC No. 44076 or PEP4-1 [Jones, Genetics, 85:12 (1977)].

Expression and cloning vectors usually contain a promoter operably linked to the Apo-3 Ligand nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:599 (1979)], alkaline phosphatase, a tryptophan (trp) promoter system [Goeddel, Nucleic Acids Res., 8:4057 (1980); EP 36, 776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding Apo-3 Ligand.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

Apo-3 Ligand transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the Apo-3 Ligand by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the Apo-3 Ligand coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding Apo-3 Ligand.

Still other methods, vectors, and host cells suitable for adaptation to the synthesis of Apo-3 Ligand in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence Apo-3 Ligand polypeptide or against a synthetic peptide based on the DNA sequences provided herein or against exogenous sequence fused to Apo-3 Ligand DNA and encoding a specific antibody epitope.

### 5. Purification of Polypeptide

Forms of Apo-3 Ligand may be recovered from culture medium or from host cell lysates. If membrane-bound, it can be released from the membrane using a suitable detergent solution (*e.g.* Triton-X 100) or by enzymatic cleavage. Cells employed in expression of Apo-3 Ligand can be disrupted by various physical or chemical means, such as freeze-thaw cycling, sonication, mechanical disruption, or cell lysing agents.

It may be desired to purify Apo-3 Ligand from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the Apo-3 Ligand. Various methods of protein purification may be employed and such methods are known in the art and described for example in Deutscher, Methods in Enzymology, 182 (1990); Scopes, Protein Purification:Principles and Practice, Springer-Verlag, New York (1982). The purification step(s) selected will depend, for example, on the nature of the production process used and the particular Apo-3 Ligand produced.

### D. Uses for Apo-3 Ligand

Nucleotide sequences (or their complement) encoding Apo-3 Ligand have various applications in the art of molecular biology, including uses as hybridization probes, in chromosome and gene mapping and in the generation of anti-sense RNA and DNA. Apo-3 Ligand nucleic acid will also be useful for the preparation of Apo-3 Ligand polypeptides by the recombinant techniques described herein.

Screening assays can be designed to find lead compounds that mimic the biological activity of a native Apo-3 Ligand or a ligand or receptor for Apo-3 Ligand. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode Apo-3 Ligand can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding Apo-3 Ligand can be used to clone genomic DNA encoding Apo-3 Ligand in accordance with established techniques and the genomic sequences used to generate transgenic animals that contain cells which express DNA encoding Apo-3 Ligand. Methods for generating transgenic animals, particularly animals such as mice or rats, have become conventional in the art and are described, for example, in U.S. Patent Nos. 4,736,866 and 4,870,009. Typically, particular cells would be targeted for Apo-3 Ligand transgene incorporation with tissue-specific enhancers. Transgenic animals that include a copy of a transgene encoding Apo-3 Ligand introduced into the germ line of the animal at an embryonic stage can be used to examine the effect of increased expression of DNA encoding Apo-3 Ligand. Such animals can be used as tester animals for reagents thought to confer protection from, for example, pathological conditions associated with its overexpression. An animal may be treated with the reagent and a reduced incidence of the pathological condition, compared to untreated animals bearing the transgene, would indicate a potential therapeutic intervention for the pathological condition.

As described herein, it was found that Apo-3 Ligand induces apoptosis in various cancer cells. Accordingly, in one embodiment of the invention, there are provided methods of inducing apoptosis in mammalian cancer cells. Among these methods are methods of administering Apo-3 Ligand to mammals to treat cancer. Suitable carriers for Apo-3 Ligand, for instance, and their formulations, are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of the carrier include buffers such as saline, Ringer's solution and dextrose solution. The pH of the solution is preferably from about 5 to about 8, and more preferably from about 7.4 to about 7.8. Further carriers include sustained release preparations such as semipermeable matrices of solid hydrophobic polymers, which matrices are in the form of shaped articles, e.g., films, liposomes or microparticles. It will be apparent to those persons skilled in the art that certain carriers may be more preferable depending upon, for instance, the route of administration and concentration of the Apo-3 Ligand molecule being administered.

Administration to a mammal may be accomplished by injection (e.g., intravenous, intraperitoneal, subcutaneous, intramuscular), or by other methods such as infusion that ensure delivery to the bloodstream in an effective form.

Effective dosages and schedules for administration may be determined empirically, and making such determinations is within the skill in the art.

In methods of treating cancer using the Apo-3 Ligand described herein, it is contemplated that other, additional therapies may be administered to the mammal, and such includes but is not limited to, chemotherapy and radiation therapy, immunoadjuvants, cytokines, and antibody-based therapies. Examples include interleukins (e.g., IL-1, IL-2, IL-3, IL-6), leukemia inhibitory factor, interferons, TGF-beta, erythropoietin, thrombopoietin, HER-2 antibody and anti-CD20 antibody. Other agents known to induce apoptosis in mammalian cells may also employed, and such agents include TNF-α, TNF-β (lymphotoxin-α), CD30 ligand, and 4-1BB ligand.

Chemotherapies contemplated by the invention include chemical substances or drugs which are known in the art and are commercially available, such as Doxorubicin, 5-Fluorouracil, Cytosine arabinoside ("Ara-C"), Cyclophosphamide, Thiotepa, Busulfan, Cytoxin, Taxol, Methotrexate, Cisplatin, Melphalan, Vinblastine and Carboplatin. Preparation and dosing schedules for such chemotherapy may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapy is preferably administered in a pharmaceutically-acceptable carrier, such as those described above. The Apo-3 Ligand may be administered sequentially or concurrently with the one or more other therapeutic agents. The amounts of Apo-3 Ligand and therapeutic agent depend, for example, on what type of drugs are used, the cancer being treated, and the scheduling and routes of administration but would generally be less than if each were used individually.

Following administration of Apo-3 Ligand to the mammal, the mammal's cancer and physiological condition can be monitored in various ways well known to the skilled practitioner. For instance, tumor mass may be observed physically or by standard x-ray imaging techniques.

### E. Anti-Apo-3 Ligand Antibodies

Anti-Apo-3 Ligand antibodies include polyclonal, monoclonal, humanized, bispecific, and heteroconjugate antibodies.

### 1. Polyclonal Antibodies

The Apo-3 Ligand antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the Apo-3 Ligand polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The Apo-3 Ligand antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the Apo-3 Ligand polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or PRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63] .

The culture medium in which the hybridoma cells are cultured can then be assayed for the presence of monoclonal antibodies directed against Apo-3 Ligand. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

In vitro methods are also suitable for preparing monovalent antibodies. Digestion of antibodies to produce fragments thereof, particularly, Fab fragments, can be accomplished using routine techniques known in the art.

### 3. Humanized Antibodies

The Apo-3 Ligand antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally; a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody.

Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)].

### 4. Bispecific Antibodies

Bispecific antibodies are monoclonal, preferably human or humanized, antibodies that have binding specificities for at least two different antigens. In the present case, one of the binding specificities is for the Apo-3 Ligand, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

### 5. Heteroconjugate Antibodies

Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### G. Uses for Apo-3 Ligand Antibodies

Anti-Apo-3 Ligand antibodies have various utilities. For example, Apo-3 Ligand antibodies may be used in diagnostic assays for Apo-3 Ligand, e.g., detecting its expression in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982).

Apo-3 Ligand antibodies also are useful for the affinity purification of Apo-3 Ligand from recombinant cell culture or natural sources. In this process, the antibodies against Apo-3 Ligand are immobilized on a suitable support, such a Sephadex resin or filter paper, using methods well known in the art. The immobilized antibody then is contacted with a sample containing the Apo-3 Ligand to be purified, and thereafter the support is washed with a suitable solvent that will remove substantially all the material in the sample except the Apo-3 Ligand, which is bound to the immobilized antibody.

Finally, the support is washed with another suitable solvent that will release the Apo-3 Ligand from the antibody.

Some of the following examples illustrate the invention; others provide useful background.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Isolation of cDNA clones Encoding Human Apo-3 Ligand

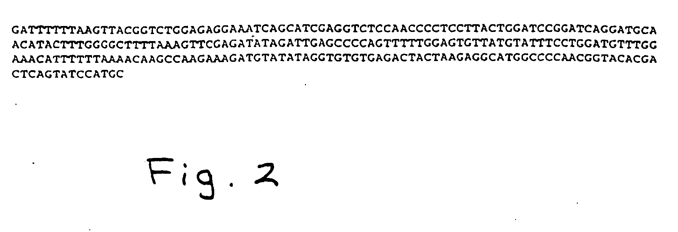

An expressed sequence tag (EST) DNA database (LIFESEQ^{™}, Incyte Pharmaceuticals, Palo Alto, CA) was searched and an EST was identified which showed homology to human Apo-2 ligand. The EST sequence is shown in Figure 2 (SEQ ID NO:3). A human fetal kidney cDNA library was then screened. mRNA isolated from human fetal kidney tissue (Clontech) was used to prepare the cDNA library. This RNA was used to generate an oligo dT primed cDNA library in the vector pRKSD using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites. The library was screened by hybridization with a synthetic oligonucleotide probe: CCAGCCCTCTGCGCTACAACCGCCAGATCGGGGAGTTTATAGTCACCCGG (SEQ ID NO:4) based on the EST.

A cDNA clone was sequenced in entirety. A nucleotide sequence of Apo-3 Ligand is shown in Figure 1 (SEQ ID NO:2). Clone DNA30879-1152 contains a single open reading frame with an apparent translational initiation site at nucleotide positions 92-94 [Kozak et al., supra] (Fig. 1; SEQ ID NO:2). The predicted polypeptide precursor is 249 amino acids long and has a calculated mass of approximately 27 kDa. Hydropathy analysis suggests a type II transmembrane protein typology, with a putative cytoplasmic region (amino acids 1-19); transmembrane region (amino acids 20-46); and extracellular region (amino acids 47-249) (see Figure 1). A potential N-linked glycosylation site appears at amino acid position 139 in the sequence of SEQ ID NO:1. Clone DNA30879-1152 has been deposited with ATCC and is assigned ATCC deposit no. 209358. Apo-3 Ligand polypeptide is obtained or obtainable by expressing the molecule encoded by the cDNA insert of the deposited ATCC 209358 vector. Digestion of the vector with XbaI and NotI will yield two inserts: 446 bp and 959 bp.

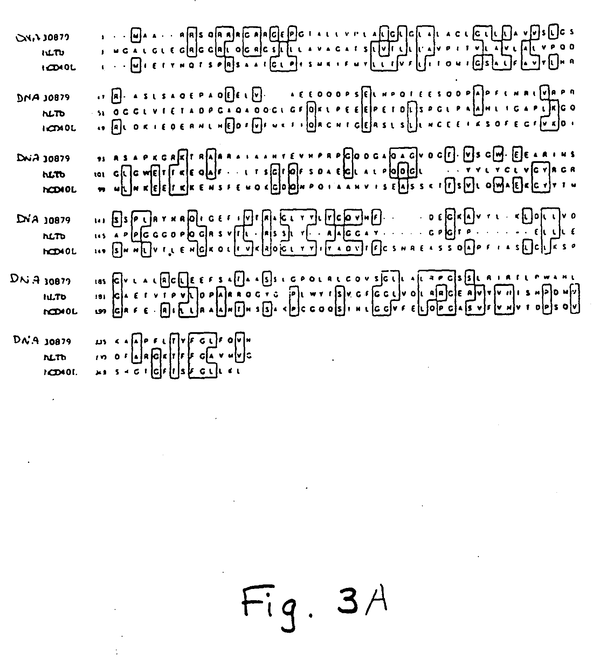

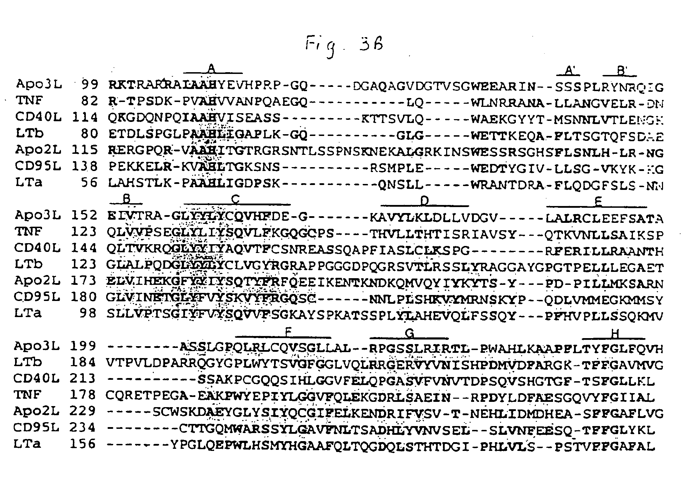

Based on a BLAST and FastA sequence alignment analysis (using the ALIGN computer program) of the full-length sequence, Apo-3 Ligand shows amino acid sequence identity to several members of the TNF cytokine family, and particularly, to human lymphotoxin-beta (23.4%) and human CD40 ligand (19.8%) (see Figure 3A). In an alignment analysis of the C-terminal ECD sequence, Apo-3 Ligand shows the highest amino acid sequence identity to TNF-alpha (22.5%); certain amino acid sequence identity was also found to CD40L (21.2%), LT-beta (20.5%), Apo-2L (19.9%), LT-alpha (15.2%), and CD95L (13.9%) (see Figure 3B). Most of the homologous amino acids are found in regions that correspond to beta-strands in the crystal structures of TNF-alpha and LT-alpha [see, Eck and Sprang, J. Biol. Chem., 264:17595-17605 (1989); Eck et al., J. Biol. Chem., 267:2119-2122 (1992)].

### EXAMPLE 2

### Northern Blot Analysis

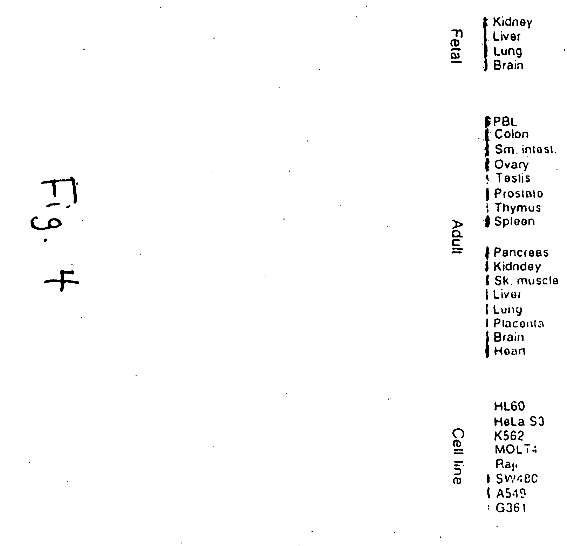

Expression of Apo-3 Ligand mRNA in human tissues and tumor cell lines was examined by Northern blot analysis (see Figure 4). Human RNA blots were hybridized to a ³²P-labelled DNA probe generated by PCR using primers based on the sequence encoding an extracellular region of the Apo-3 Ligand polypeptide (amino acids 47 to 249 of Fig. 1):
CGACGACAAGCATATGCGGGCATCGCTGTCCGCCCAGGAG (SEQ ID NO:5);
CAGCCGGATCCTCGAGTCAGTGAACCTGGAAGAGTCCG (SEQ ID NO:6). Human fetal, adult, or cancer cell line mRNA blots (Clontech) were incubated with the DNA probe in hybridization buffer (5X SSPE; 2X Denhardt's solution; 100 mg/mL denatured sheared salmon sperm DNA; 50% formamide; 2% SDS) for 60 hours at 42°C. The blots were washed several times in 2X SSC; 0.05% SDS for 1 hour at room temperature, followed by a 30 minute wash in 0.1X SSC; 0.1% SDS at 50°C. The blots were developed after overnight exposure by phosphorimager analysis (Fuji).

As shown in Fig. 4, a single mRNA transcript of about 2 kb was detected. This transcript was expressed in fetal kidney, liver, lung, and brain, and in many adult tissues, particularly in ovary, spleen and heart. Expression was also detected in the following human tumor cell lines: G361 melanoma, A549 lung carcinoma, SW480 colon carcinoma, and HeLa S3 cervical carcinoma.

### EXAMPLE 3

### Expression of Apo-3 Ligand in E. coli

The DNA sequence (of Fig. 1; SEQ ID NO:2) encoding an extracellular region of the Apo-3 Ligand polypeptide (amino acids 47 to 249 of Fig. 1; SEQ ID NO:1) was amplified with PCR primers (see Example 2 primers) and subcloned into the plasmid pET19B (Novagen) downstream and in frame of a Met Gly His₁₀ sequence followed by a 12 amino acid enterokinase cleavage site (derived from the plasmid): Met Gly His His His His His His His His His His Ser Ser Gly His Ile Asp Asp Asp Asp Lys His Met (SEQ ID NO: 7).

The resulting plasmid was used to transform *E. coli* strain JM109 (ATCC 53323) using the methods described in Sambrook et al., supra. Transformants were identified by PCR. Plasmid DNA was isolated and confirmed by restriction analysis and DNA sequencing.

Selected clones were grown overnight in liquid culture medium LB supplemented with antibiotics. The overnight culture was subsequently used to inoculate a larger scale culture. The cells were grown to a desired optical density, during which the expression promoter is turned on.

After culturing the cells for several more hours, the cells were harvested by centrifugation. The cell pellet obtained by the centrifugation was solubilized using a microfluidizer in a buffer containing 0.1M Tris, 0.2M NaCl, 50 mM EDTA, pH 8.0. The solubilized Apo-3 Ligand protein was purified using Nickel-sepharose affinity chromatography.

### EXAMPLE 4

### Activation of NF-κB by Soluble Apo-3 Ligand

Several assays were conducted to determine whether soluble Apo-3 Ligand activates NF-κB.

In a first assay, HeLa cells (ATCC CCL 22) were treated with His-tagged soluble Apo-3 Ligand (described in Example 3) (10 µg/ml) in duplicate or with vehicle for 30 minutes. His-tagged Apo-2 ligand (see WO 97/25428) or TNF-alpha (Pennica et al., Nature, 312:724-729 (1984)) were assayed for comparison. Nuclear extracts were prepared and 1 µg of nuclear protein was reacted with a ³²P-labelled NF-κB-specific synthetic oligonucleotide probe ATCAGGGACTTTCCGCTGGGGACTTTCCG (SEQ ID NO:8) [see, also, MacKay et al., J. Immunol., 153:5274-5284 (1994)].

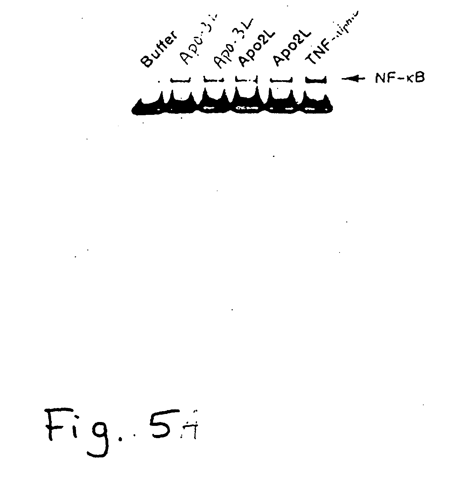

The results are shown in Fig. 5A. As shown in Fig. 5A, the soluble Apo-3 Ligand polypeptide induced significant NF-κB activation in HeLa cells as measured by an electrophoretic mobility shift assay [Marsters et al., Proc. Natl. Acad. Sci., 92:5401-5405 (1995)]; the level of activation was comparable to activation observed for Apo-2 ligand, but weaker than activation by TNF-alpha.

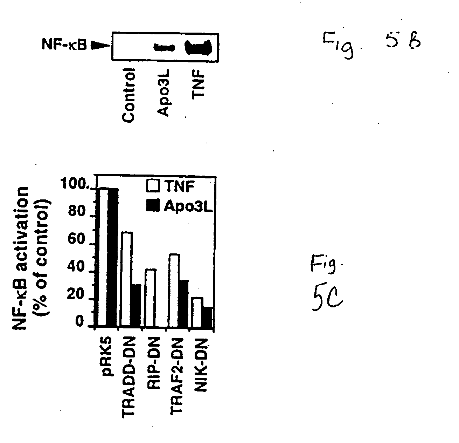

In another assay, human 293 cells (ATCC CCL 1573) were treated as above (except the cells were incubated with the respective ligands for 3 hours) and NF-κB activation was determined in the electrophoretic mobility shift assay as above. The results are shown in Figure 5B.

The 293 cells were also tested in an assay in which the cells were transfected by calcium phosphate precipitation with empty vector (pRK5; EP 307,247) or with pRK5 expression plasmids encoding dominant-negative (DN) mutants of TRADD, RIP, TRAF2, or NIK (Tularik, South San Francisco, CA). After a 16 hour incubation, the cells were treated with soluble Apo-3 Ligand or TNF-alpha and assayed for NF-κB activation as above. The results are shown in Figure 5C.

### EXAMPLE 5

### Chromosomal Localization of the Apo-3 Ligand gene

Chromosomal localization of the Apo-3 Ligand gene was examined by radiation hybrid (RH) panel analysis. RH mapping was performed by PCR using a human-mouse cell radiation hybrid panel (Research Genetics) and primers based on the coding region of the Apo-3 Ligand cDNA:
CCGCAGTCGTCCCAGGCTGCCGGC (SEQ ID NO:9) and
GGAGCTAGTGAGGTGGAGATGGG (SEQ ID NO:10) [Gelb et al., Hum. Genet., 98:141 (1996)]. Analysis of the PCR data using the Stanford Human Genome Center Database and the Whitehead Institute for Biomedical Research/MIT Center for Genome Research indicated that Apo-3 Ligand is linked to the marker SHGC-31370, with an LOD of 6.8, which maps to human chromosome 17p12-13. This analysis also showed that Apo-3 Ligand is closely linked to the genomic locus of the p53 tumor suppressor.

### EXAMPLE 6

### Apoptotic Activity of Full length Apo-3 Ligand

A pRK5 plasmid (see EP 307,247) encoding Apo-3 Ligand polypeptide (amino acids 1 to 249 of Fig. 1), or empty pRK5 plasmid, was transiently transfected along with a pRK5 plasmid encoding human CD4 as a marker for transfection into human HeLa cells by electroporation, and the cells were plated in tissue culture dishes.

Four hours later, the caspase inhibitor z-VAD-fmk (Research Biochemicals) (200 µM) was added to some of the dishes. Twenty hours later, apoptosis was determined by FACS analysis of cells positive for the CD4 marker by measuring binding of fluorescein isothiocyanate (FITC)-conjugated annexin V (Brand Applications) (see WO 97/25428).

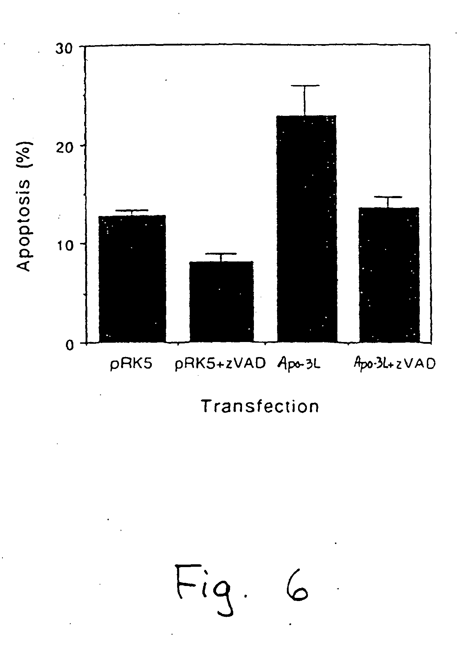

As shown in Figure 6, transfection by Apo-3 Ligand resulted in about a doubling of the level of apoptosis as compared with pRK5 (control). This increase in apoptosis was blocked by z-VAD-fmk, confirming the involvement of caspases in this effect.

### EXAMPLE 7

### Activation of Jun N-terminal kinase by Full length Apo-3 Ligand

The pRK5 plasmid encoding Apo-3 Ligand polypeptide or empty pRK5 plasmid (see Example 6 above) was transiently transfected into human 293 cells (ATCC CCL 1573) by calcium phosphate precipitation. Four hours later, one set of cells was treated with z-VAD-fmk (Research Biochemicals) (200 µM). Twenty hours later, the cells were harvested and analyzed for activity of Jun N-terminal kinase (JNK; also called stress activated protein kinase or SAPK) using a commercially available JNK/SAPK assay kit (New England Biolabs), and according to the manufacturer's instructions.

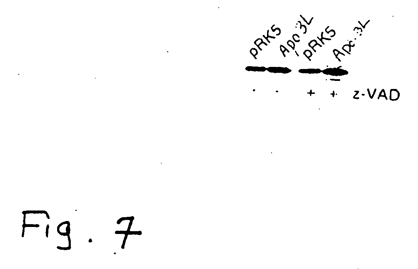

As shown in Figure 7, transfection by Apo-3 Ligand resulted in a detectable increase in the level of JNK/SAPK activity as compared with the pRK5 control. Treatment with z-VAD-fmk augmented JNK activation by Apo-3 Ligand, suggesting that JNK activation by Apo-3 Ligand is enhanced when apoptosis activation is prevented by z-VAD-fmk.

### EXAMPLE 8

### Binding of Apo-3 by Apo-3 Ligand

A binding assay was conducted to determine if Apo-3 Ligand binds Apo-3 receptor. Apo-3 receptor was described by Marsters et al., Curr. Biol., 6:750 (1996). Other investigators have also referred to Apo-3 as DR3 [Chinnayian et al., Science, 274:990 (1996)], Ws1-1 [Kitson et al., Nature, 384:372 (1996)], TRAMP [Bodmer et al., Immunity, 6:79 (1997)], or LARD [Screaton et al., PNAS, 94:4615-4619 (1997)].

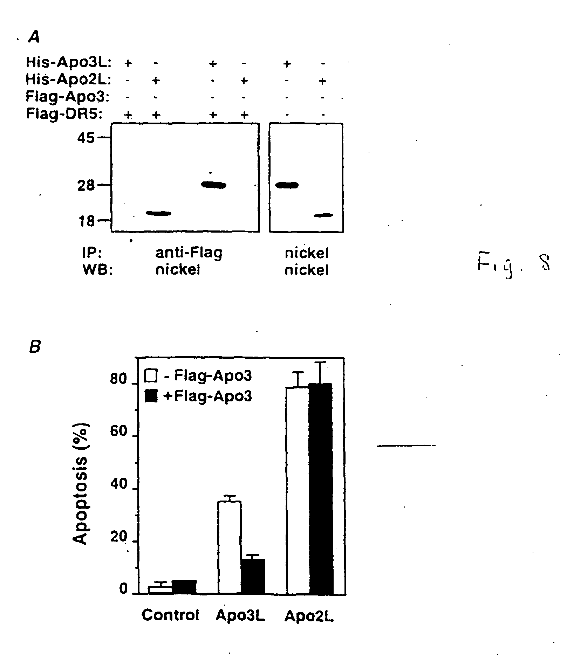

A Flag-epitope tagged soluble Apo-3 was produced (as described for DR5 in Sheridan et al., Science, 277:818 (1997)) and tested in a co-immunoprecipitation assay to examine its ability to associate with a histidine tagged soluble Apo-3 Ligand (see Example 3 above). For comparison, histidine-tagged Apo-2L [prepared as described in Pitti et al., J. Biol. Chem., 271:12687 (1996)] and the ECD of the Apo-2L receptor, DR5 [prepared as described in Sheridan et al., Science, 277:818 (1997); see also, Pan et al., Science, 277:815 (1997)] were also tested in the assay. The respective ligands (1 µg/ml) and receptors (1 µg/ml) were incubated for 1 hour at room temperature. The reaction mixtures were subjected to precipitation with anti-Flag antibody conjugated to sepharose beads (Kodak) (Figure 8A, left) or nickel-sepharose (Qiagen) (Figure 8A, right) by overnight incubation at 4°C according to manufacturer's instructions and resolved by gel electrophoresis. The ligands were visualized by Western blot analysis with nickel-conjugated horseradish peroxidase. In Figure 8A, molecular weight markers (kDa) are indicated on the left.

The data revealed that Apo-3 Ligand bound to Apo-3 but not to DR5, and that Apo-2 Ligand bound to DR5, but not to Apo-3. Thus, there appeared to be a specific complex formation between Apo-3 Ligand and Apo-3. Further, preincubation of ligand with Flag tagged Apo-3 and immunodepletion of complexes with anti-Flag antibody-conjugated sepharose beads as above inhibited apoptosis induction by Apo-3 Ligand, but not by Apo-2L (see Figure 8B), supporting the conclusion that Apo-3 Ligand binds to Apo-3.

### EXAMPLE 9

### Apoptotic Activity of Soluble Apo-3 Ligand

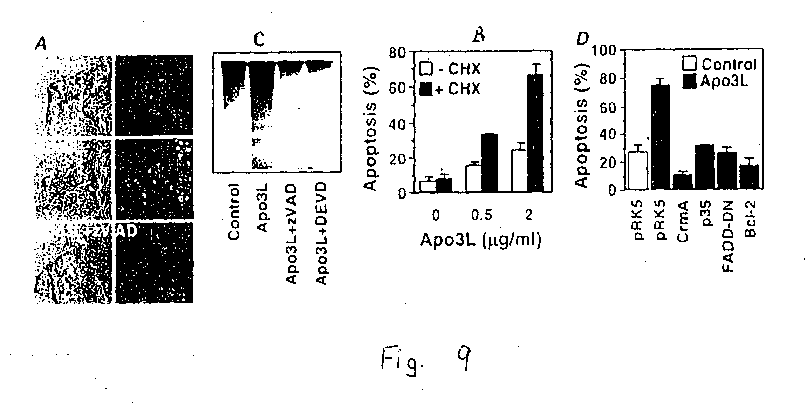

Human MCF-7 breast carcinoma cells (ATCC HTB 22) were pre-treated for 1 hour with cyclohexamide (CHX) (10 µg/ml) and incubated for 14 hours with soluble Apo-3 Ligand (2 µg/ml; prepared as described in Example 3 above) alone or together with zVAD-fmk (Research Biochemicals) (100 nM). The cells were then stained with Hoechst dye and photographed by phase (Figure 9A, left) and by fluorescence (Figure 9A, right) microscopy. In Figure 9A, arrowheads indicate some of the apoptotic cells (left) and their condensed nucleic (right).

The soluble Apo-3 Ligand induced marked apoptosis in the MCF-7 cells within a period of 14 hours (see Figure 9A) as evidenced by the morphological changes and chromatin condensation.

In a separate experiment, the MCF-7 cells were pre-treated for 1 hour with buffer or CHX, incubated for an additional 14 hours with the indicated concentrations of soluble Apo-3 Ligand (see Figure 9B), and apoptotic or live cells were score by microscopy. Apo-3 ligand-induced apoptosis was augmented by the translation inhibitor, cyclohexamide, indicating that this response is independent of de novo protein synthesis (see Figure 9B).

HeLa S3 cervical carcinoma cells (ATCC CCL 2.2) were similarly pre-treated with CHX and then incubated with soluble Apo-3 Ligand (prepared as described in Example 3 above) alone or together with zVAD-fmk or DEVD-fmk (Research Biochemicals) (100nM). The treated cells were then subjected to DNA fragmentation analysis. The soluble Apo-3 Ligand induced marked apoptosis in the HeLa S3 cells (see Figure 9C) as evidenced by internucleosomal DNA fragmentation. Addition of the caspase inhibitors, DEVD-fmk and zVAD-fmk), or transfection with the virus-derived caspase inhibitors CrmA or p35 (see below, Figure 9D) blocked induction of apoptosis by Apo-3 Ligand, indicating a requirement for caspase activity in this response.

MCF-7 cells were transfected by lipofection with empty vector (pRK5; EP 307,247) or with expression plasmid encoding the caspase inhibitors, p35 or CrmA, or a dominant-negative FADD mutant (Tularik, South San Francisco, CA). Sixteen hours later, the cells were pre-treated with CHX for 1 hour, and then treated for an additional 14 hours with soluble Apo-3 Ligand (2 µg/ml).

The results are shown in Figure 9D. Transfection with a dominant-negative mutant of FADD, which contains the adaptor's death domain [see, Hsu et al.; Cell, 84:299 (1996)], prevented apoptosis induction by Apo-3 Ligand. Thus, Apo-3 Ligand appears to signal cell death through FADD itself or through a closely related protein.

### EXAMPLE 10

### Expression of Apo-3 Ligand in mammalian cells

This example illustrates preparation of Apo-3 Ligand by recombinant expression in mammalian cells.

The vector, pRK5 (see EP 307,247, published March 15, 1989), is employed as the expression vector. Optionally, the Apo-3 Ligand DNA is ligated into pRK5 with selected restriction enzymes to allow insertion of the Apo-3 Ligand DNA using ligation methods such as described in Sambrook et al., supra. The resulting vector is called pRK5-Apo-3 Ligand.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-Apo-3 Ligand DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of Apo-3 Ligand polypeptide. The cultures containing transfected cells may undergo further incubation (in serum free medium) and the medium is tested in selected bioassays.

In an alternative technique, Apo-3 Ligand may be introduced into 293 cells transiently using the dextran sulfate method described by Sompraryrac et al., Proc. Natl. Acad. Sci., 12:7575 (1981). 293 cells are grown to maximal density in a spinner flask and 700 µg pRK5-Apo-3 Ligand DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed Apo-3 Ligand can then be concentrated and purified by any selected method, such as dialysis and/or column chromatography.

In another embodiment, Apo-3 Ligand can be expressed in CHO cells. The pRK5-Apo-3 Ligand can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of Apo-3 Ligand polypeptide, the culture medium may be replaced with serum free medium. Preferably, the cultures are incubated for about 6 days, and then the conditioned medium is harvested. The medium containing the expressed Apo-3 Ligand can then be concentrated and purified by any selected method.

Epitope-tagged Apo-3 Ligand may also be expressed in host CHO cells. The Apo-3 Ligand may be subcloned out of the pRK5 vector. The subclone insert can undergo PCR to fuse in frame with a selected epitope tag such as a poly-his tag into a Baculovirus expression vector. The poly-his tagged Apo-3 Ligand insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged Apo-3 Ligand can then be concentrated and purified by any selected method, such as by Ni²⁺-chelate affinity chromatography.

### EXAMPLE 11

### Expression of Apo-3 Ligand in Yeast

The following method describes recombinant expression of Apo-3 Ligand in yeast.

First, yeast expression vectors are constructed for intracellular production or secretion of Apo-3 Ligand from the ADH2/GAPDH promoter. DNA encoding Apo-3 Ligand, a selected signal peptide and the promoter is inserted into suitable restriction enzyme sites in the selected plasmid to direct intracellular expression of Apo-3 Ligand. For secretion, DNA encoding Apo-3 Ligand can be cloned into the selected plasmid, together with DNA encoding the ADH2/GAPDH promoter, the yeast alpha-factor secretory signal/leader sequence, and linker sequences (if needed) for expression of Apo-3 Ligand.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant Apo-3 Ligand can subsequently be isolated and purified by removing the yeast cells from the fermentation medium by centrifugation and then concentrating the medium using selected cartridge filters. The concentrate containing Apo-3 Ligand may further be purified using selected column chromatography resins.

### EXAMPLE 12

### Expression of Apo-3 Ligand in Baculovirus

The following method describes recombinant expression of Apo-3 Ligand in Baculovirus.

The Apo-3 Ligand is fused upstream of an epitope tag contained with a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the Apo-3 Ligand or the desired portion of the Apo-3 Ligand (such as a sequence encoding an extracellular domain, e.g., amino acids 47 to 249 of Fig. 1 (SEQ ID NO:1)) is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL) . After 4 - 5 days of incubation at 28°C, the released viruses are harvested and used for further amplifications. Viral infection and protein expression is performed as described by O'Reilley et al., Baculovirus expression vectors: A laboratory Manual, Oxford:Oxford University Press (1994).

Expressed poly-his tagged Apo-3 Ligand can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% Glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% Glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% Glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or western blot with Ni²⁺-NTA-conjugated to alkaline phosphatase (Qiagen). Fractions containing the eluted His₁₀-tagged Apo-3 Ligand are pooled and dialyzed against loading buffer.

Alternatively, purification of the IgG tagged (or Fc tagged) Apo-3 Ligand can be performed using known chromatography techniques, including for instance, Protein A or protein G column chromatography.

### EXAMPLE 13

### Preparation of Antibodies that Bind Apo-3 Ligand

This example illustrates preparation of monoclonal antibodies which can specifically bind Apo-3 Ligand.

Techniques for producing the monoclonal antibodies are known in the art and are described, for instance, in Goding, supra. Immunogens that may be employed include purified Apo-3 Ligand, fusion proteins containing Apo-3 Ligand, and cells expressing recombinant Apo-3 Ligand on the cell surface. Selection of the immunogen can be made by the skilled artisan without undue experimentation.

Mice, such as Balb/c, are immunized with the Apo-3 Ligand immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect Apo-3 Ligand antibodies.

After a suitable antibody titer has been detected, the animals "positive" for antibodies can be injected with a final intravenous injection of Apo-3 Ligand. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against Apo-3 Ligand. Determination of "positive" hybridoma cells secreting the desired monoclonal antibodies against Apo-3 Ligand is within the skill in the art.

The positive hybridoma cells can be injected intraperitoneally into syngeneic Balb/c mice to produce ascites containing the anti-Apo-3 Ligand monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 14

### Use of Apo-3 Ligand as a hybridization probe

The following method describes use of a nucleotide sequence encoding Apo-3 Ligand as a hybridization probe.

DNA comprising the coding sequence of Apo-3 Ligand (as shown in Figure 1, SEQ ID NO:2) is employed as a probe to screen for homologous DNAs (such as those encoding naturally-occurring variants of Apo-3 Ligand) in human tissue cDNA libraries or human tissue genomic libraries.

Hybridization and washing of filters containing either library DNAs is performed under the following high stringency conditions. Hybridization of radiolabeled Apo-3 Ligand-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence Apo-3 Ligand can then be identified using standard techniques known in the art.

### Deposit of Material

The following materials have been deposited with the American Type Culture Collection, 10801 University Boulevard, Manassas, Virginia, USA (ATCC):

| Material | ATCC Dep. No. | Deposit Date |
|---|---|---|
| DNA30879-1152 | 209358 | October 10, 1997 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

## Claims

1. An isolated Apo-3 Ligand polypeptide consisting of amino acid residues 42 to 249, 47 to 249, or 52 to 249 of Fig. 1 (SEQ ID NO:1).

2. A chimeric molecule comprising the Apo-3 Ligand polypeptide of claim 1 fused to a heterologous amino acid sequence.

3. The chimeric molecule of claim 2 wherein said heterologous amino acid sequence is an epitope tag sequence.

4. The chimeric molecule of claim 2 wherein said heterologous amino acid sequence is an immunoglobulin sequence.

5. The chimeric molecule of claim 4 wherein said immunoglobulin sequence is an IgG Fc domain.

6. An isolated nucleic acid consisting of DNA encoding the Apo-3 Ligand polypeptide of claim 1.

7. An isolated nucleic acid comprising DNA encoding an Apo-3 Ligand polypeptide, said DNA having the sequence of SEQ ID NO:2.

8. A vector comprising the nucleic acid of claim 6 or claim 7.

9. The vector of claim 8 wherein the DNA is operably linked to control sequences recognized by a host cell transformed with the vector.

10. A host cell comprising the vector of claim 8.

11. The host cell of claim 10 wherein said cell is a CHO cell.

12. The host cell of claim 10 wherein said cell is an *E*. coli.

13. The host cell of claim 10 wherein said cell is a yeast cell.

14. A process for producing Apo-3 Ligand polypeptides comprising culturing the host cell of claim 10 under conditions suitable for expression of Apo-3 Ligand and recovering Apo-3-Ligand from the cell culture.

15. The vector deposited as ATCC 209358.

16. A method of inducing apoptosis in mammalian cancer cells comprising exposing mammalian cancer cells in vitro to an effective amount of the Apo-3 Ligand polypeptide of claim 1 or the chimeric molecule of any one of claims 2 to 5.

17. A method of inducing NF-κB-dependent gene transcription in mammalian cells comprising exposing mammalian cells in vitro to an effective amount of the Apo-3 Ligand polypeptide of claim 1 or the chimeric molecule of any one of claims 2 to 5.

18. A method of inducing JNK/SAPK-dependent responses in mammalian cells comprising exposing mammalian cells in vitro to an effective amount of the Apo-3 Ligand polypeptide of claim 1 or the chimeric molecule of any one of claims 2 to 5.

19. Use of the Apo-3 Ligand polypeptide of claim 1, or the chimeric molecule of any one of claims 2 to 5, in the preparation of a medicament for the treatment of cancer in a mammal.

## Patentansprüche

1. Isoliertes Apo-3-Ligandenpolypeptid, das aus den Aminosäureresten 42 bis 249, 47 bis 249 oder 52 bis 249 aus Fig. 1 (Seq.-ID Nr. 1) besteht.

2. Chimäres Molekül, das Apo-3-Ligandenpolypeptid nach Anspruch 1 an eine heterologe Aminosäuresequenz fusioniert umfassend.

3. Chimäres Molekül nach Anspruch 2, worin die heterologe Aminosäuresequenz eine Epitop-Markierungssequenz ist.

4. Chimäres Molekül nach Anspruch 2, worin die heterologe Aminosäuresequenz eine Immunglobulinsequenz ist.

5. Chimäres Molekül nach Anspruch 4, worin die Immunglobulinsequenz eine IgG-Fc-Domäne ist.

6. Isolierte Nucleinsäure, aus DNA bestehend, die für das Apo-3-Ligandenpolypeptid nach Anspruch 1 kodiert.

7. Isolierte Nucleinsäure, DNA umfassend, die für ein Apo-3-Ligandenpolypeptid kodiert, wobei die DNA die Sequenz der Seq.-ID Nr. 2 aufweist.

8. Vektor, die Nucleinsäure nach Anspruch 6 oder Anspruch 7 umfassend.

9. Vektor nach Anspruch 8, worin die DNA operabel an Kontrollsequenzen gebunden ist, die von einer mit dem Vektor transformierten Wirtszelle erkannt werden.

10. Wirtszelle, einen Vektor nach Anspruch 8 umfassend.

11. Wirtszelle nach Anspruch 10, worin die Zelle eine CHO-Zelle ist.

12. Wirtszelle nach Anspruch 10, worin die Zelle E. coli ist.

13. Wirtszelle nach Anspruch 10, worin die Zelle eine Hefezelle ist.

14. Verfahren zur Herstellung von Apo-3-Ligandenpolypeptiden, umfassend das Kultivieren einer Wirtszelle nach Anspruch 10 unter Bedingungen, die für die Expression eines Apo-3-Liganden geeignet sind, und das Gewinnen eines Apo-3-Liganden aus der Zellkultur.

15. Vektor, der als ATCC 209358 hinterlegt ist.

16. Verfahren zur Induktion von Apoptose in Säugetierkrebszellen, umfassend das Aussetzen von Säugetierkrebszellen in vitro gegenüber einer wirksamen Menge eines Apo-3-Ligandenpolypeptids nach Anspruch 1 oder eines chimären Moleküls nach einem der Ansprüche 2 bis 5.

17. Verfahren zur Induktion von NF-κB-abhängiger Gentranskription in Säugetierzellen, umfassend das Aussetzen von Säugetierzellen in vitro gegenüber einer wirktsamen Menge eines Apo-3-Ligandenpolypeptids nach Anspruch 1 oder eines chimären Moleküls nach einem der Ansprüche 2 bis 5.

18. Verfahren zur Induktion von JNK/SAPK-abhängigen Reaktionen in Säugetierzellen, umfassend das Aussetzen von Säugetierzellen in vitro gegenüber einer wirksamen Menge eines Apo-3-Ligandenpolypeptids nach Anspruch 1 oder eines chimären Moleküls nach einem der Ansprüche 2 bis 5.

19. Verwendung eines Apo-3-Ligandenpolypeptids nach Anspruch 1 oder eines chimären Moleküls nach einem der Ansprüche 2 bis 5 zur Herstellung eines Medikaments zur Behandlung von Krebs in einem Säugetier.

## Revendications

1. Polypeptide ligand de Apo-3 isolé, consistant en les résidus d'aminoacides 42 à 249, 47 à 249 ou 52 à 249 de la figure 1 (SEQ ID N° 1).

2. Molécule chimère comprenant le polypeptide ligand de Apo-3 de la revendication 1 fusionné à une séquence d'aminoacides hétérologue.

3. Molécule chimère suivant la revendication 2, dans laquelle ladite séquence d'aminoacides hétérologue est une séquence de marqueur d'épitope.

4. Molécule chimère suivant la revendication 2, dans laquelle ladite séquence d'aminoacidés hétérologue est une séquence d'immunoglobuline.

5. Molécule chimère suivant la revendication 4, dans laquelle ladite séquence d'immunoglobuline est un domaine Fc d'une IgG.

6. Acide nucléique isolé consistant en un ADN codant pour le polypeptide ligand de Apo-3 de la revendication 1.

7. Acide nucléique isolé comprenant un ADN codant pour un polypeptide ligand de Apo-3, ledit ADN ayant la séquence de la SEQ ID N° 2.

8. Vecteur comprenant l'acide nucléique de la revendication 6 ou de la revendication 7.

9. Vecteur suivant la revendication 8, dans lequel l'ADN est lié de manière fonctionnelle à des séquences de régulation reconnues par une cellule hôte transformée avec le vecteur.

10. Cellule hôte comprenant le vecteur de la revendication 8.

11. Cellule hôte suivant la revendication 10, ladite cellule étant une cellule CHO.

12. Cellule hôte suivant la revendication 10, ladite cellule étant une cellule de *E*. *coli.*

13. Cellule hôte suivant la revendication 10, ladite cellule étant une cellule de levure.

14. Procédé pour la production de polypeptides ligands de Apo-3, comprenant la culture de la cellule hôte de la revendication 10 dans des conditions convenables pour l'expression du ligand de Apo-3 et la récupération du ligand de Apo-3 à partir de la culture cellulaire.

15. Vecteur déposé sous la référence ATCC 209358.

16. Méthode pour induire une apoptose dans les cellules cancéreuses d'un mammifère, comprenant l'exposition de cellules cancéreuses d'un mammifère in vitro à une quantité efficace du polypeptide ligand de Apo-3 de la revendication 1 ou de la molécule chimère de l'une quelconque des revendications 2 à 5.

17. Méthode pour induire une transcription de gène dépendant de NF-κB dans des cellules de mammifère, comprenant l'exposition de cellules de mammifère in vitro à une quantité efficace du polypeptide ligand de Apo-3 de la revendication 1 ou de la molécule chimère de l'une quelconque des revendications 2 à 5.

18. Méthode pour induire des réponses dépendant de JNK/SAPK dans des cellules de mammifère, comprenant l'exposition de cellules de mammifère in vitro à une quantité efficace du polypeptide ligand de Apo-3 de la revendication 1 ou de la molécule chimère de l'une quelconque des revendications 2 à 5.

19. Utilisation du polypeptide ligand de Apo-3 de la revendication 1, ou de la molécule chimère de l'une quelconque des revendications 2 à 5, dans la préparation d'un médicament destiné au traitement du cancer chez un mammifère.
